# EUROPÄISCHE PATENTSCHRIFT

(11) **EP 0 842 191 B1**
(45) Veröffentlichungstag und Bekanntmachung des Hinweises auf die Patenterteilung: **21.11.2001**
(21) Anmeldenummer: 96927023.0
(22) Anmeldetag: 17.07.1996
(51) Int. Cl.: C07K 7/64, C12P 21/04, A61K 38/13

(54) **CYCLOSPORIN-DERIVATE MIT ANTI-HIV-WIRKUNG**
CYCLOSPORIN DERIVATIVES WITH ANTI-HIV ACTIVITY
DERIVES DE CYCLOSPORIN AYANT UNE ACTIVITE CONTRE HIV

(30) Priorität: 17.07.1995 EP 95111162
(43) Veröffentlichungstag der Anmeldung: 20.05.1998
(73) Patentinhaber: C-Chem AG, 4102 Binningen (CH)
(72) Erfinder: LÜCHINGER, Jean, M., CH-4059 Basel (CH)
(74) Vertreter: Kunz, Ekkehard, Dr.
(86) Internationale Anmeldenummer: EP9603129
(87) Internationale Veröffentlichungsnummer: WO9704005

(56) Entgegenhaltungen:
- EP-A- 0 194 972
- EP-A- 0 444 897
- EP-A- 0 484 281
- BIOORGANIC AND MEDICINAL CHEMISTRY LETTERS, Bd. 6, Nr. 1, 1996, Seiten 23-26, XP000615812 C. PAPAGEORGIOU ET AL.: "Anit HIV-1 activity of a hydrophilic cyclosporin derivative with improved affinity to cyclophilin"
- BIOORGANIC AND MEDICINAL CHEMISTRY LETTERS, Bd. 6, Nr. 4, 1996, Seite 497 XP002022422 C. PAPAGEORGIOU ET AL.: "Anti HIV-1 activity of a hydrophilic cyclosporin derivative with improved affinity to cyclophilin"
- J. VIROL. (1995), 69(4), 2451-61 CODEN: JOVIAM;ISSN: 0022-538X, April 1995, XP002022423 BILLICH, ANDREAS ET AL: "Mode of action of SDZ NIM 811, a nonimmunosuppressive cyclosporin A analog with activity against human immunodeficiency virus (HIV) type 1: interference with HIV protein-cyclophilin A interactions"
- HELV. CHIM. ACTA (1993), 76(4), 1564-90 CODEN: HCACAV;ISSN: 0018-019X, 1993, XP002022424 SEEBACH, DIETER ET AL: "Modification of cyclosporin A (CS): generation of an enolate at the sarcosine residue and reactions with electrophiles"

## Beschreibung

Die vorliegende Erfindung betrifft neue cyclische Peptide aus der Cyclosporin-Reihe mit starker Hemmwirkung gegen das Humane Immunodefizienzvirus (HIV) ohne immunsuppressive Aktivität. Solche cyclischen Peptide sind unter anderem in EP 484 281 beansprucht. Eine der in dieser Patentschrift konkret beanspruchten Substanzen ist (gamma-Hydroxy-N-methylleucin)-cyclosporin. Diese Substanz ist sehr einfach aus Cyclosporin A durch mikrobiologische Hydroxylierung herstellbar, jedoch ist in EP 484 281 die Aktivität dieser Substanz gegen HIV als etwa 5-6 mal schwächer angegeben, als jene der am stärksten aktiven Substanzen, nämlich MeIle-4-cyclosporin oder MeVal-4-cyclosporin. Alle 3 Substanzen haben praktisch keine immunsuppressive Aktivität. Unerwarteterweise wurde gefunden, dass durch Einführung geeigneter Substituenten in die Methylengruppe der Aminosäure Sarcosin in Position 3 von (gamma-Hydroxy-MeLeu-4)-cyclosporin dessen anti-HIV Wirkung erheblich verbessert werden kann, ohne immunsuppresive Aktivitäten zu erzeugen. Da die therapeutisch verwendeten Dosen von Cyclosporin A, die nach Organtransplantationen angewandt werden, um die Abstossung der transplantierten Organe zu verhindern, sehr hoch sind, und für eine anti-HIV Therapie mit Cyclosporin-Derivaten ähnlich hohe Dosen zu erwarten sind, besteht der Wert der vorliegenden Erfindung darin, dass neue Cyclosporin-Derivate mit hoher anti-HIV Aktivität bereitgestellt werden, und dass deren Herstellung gleichzeitig in wenigen Schritten aus Cyclosporin A, einem bereits im Tonnenmasstab hergestellten Produkt erfolgen kann.

Gegenstand der vorliegenden Erfindung sind demnach neue cyclische Peptide der allgemeinen Formel 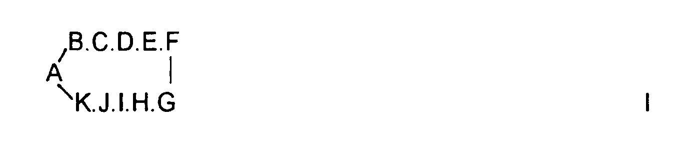 worin die Buchstaben A bis K Reste der folgenden Aminosäuren bedeuten:
- A: ein substituiertes Homothreonin der allgemeinen Formel

R₁-CH₂ CH(CH₃)-CH(OH)-CH(NHCH₃)-COOH II

in welcher R₁ n-Propyl oder Propenyl bedeutet und die Doppelbindung vorzugsweise trans konfiguriert ist,
- B: alpha-Aminobuttersäure, Valin, Norvalin, oder Threonin,
- C: eine (D)-Aminosäure der allgemeinen Formel

CH₃NH-CH(R)-COOH III

in der
R= C₂ - C₆ Alkyl, Alkenyl, Alkinyl, geradkettig oder verzweigt, wobei diese Gruppen noch durch Hydroxy, Amino, C₁-C₄ Alkylamino, C₁-C₄ Dialkylamino, Alkoxy oder Acyloxy substituiert sein können,
COOR₂, CONHR₂, wobei R₂ = C₁-C₄ Alkyl, geradkettig oder verzweigt sein kann,
X-R₃, wobei X = O, S, und R₃ = C₁-C₄ Alkyl, Alkenyl, Alkinyl, geradkettig oder verzweigt, und wobei wenn X = S, R₃ = auch Aryl oder Heteroaryl sein kann,
Halogen, vorzugsweise Fluor,
Cyano,
CHR₄R₅, wobei R₄ = Wasserstoff, Methyl, Ethyl, Phenyl, und R₅ = Wasserstoff, Hydroxy, Halogen, vorzugsweise Fluor, weiters Amino, C₁- C₄ Alkylamino, C₁-C₄ Dialkylamino, Acyloxy, vorzugsweise Acetyloxy, weiters tert-Butoxycarbonyl-aminoethoxy-ethoxy-acetyloxy, Alkoxycarbonyl, vorzugsweise Butoxycarbonyl,
- D: N-Methyl-gamma-hydroxy-leucin oder N-Methyl-gamma-acetyloxyleucin,
- E: Valin,
- F, I, und J: jeweils N-Methylleucin,
- G: Alanin,
- H: (D)-Alanin, (D)-Serin, und
- K: N-Methyl-Valin.

Ein wesentliches Element der vorliegenden Erfindung ist die Variation des Restes R in Formel III der Aminosaeure C.
Beispiele für den Rest R in Formel III sind C₁ - C₆ Alkyl, Alkenyl, Alkinyl, geradkettig oder verzweigt, wobei diese Gruppen noch durch Hydroxy, Alkyloxy, Acyloxy, C₁-C₄ Alkylamino, C₁-C₄ Dialkylamino, substituiert sein können. Beispiele hiefür sind Methyl, Ethyl, Propyl, Isopropyl, n-Butyl, Isobutyl, tertiär-Butyl, Pentyl, Isopentyl, Neopentyl, tertiaer-Pentyl, Hexyl, Cyclopropylmethyl, Allyl, Butenyl, Pentenyl, Isopentenyl, Propargyl, Butinyl. Beispiele für die Alkyloxy und Acyloxy Substituenten im Rest R sind etwa Methoxy, Ethoxy, Propyloxy, beta-Methoxyethoxy, sowie Acetoxy oder Pivaloyloxy. Beispiele für die C₁-C₄ Alkylamino und C₁-C₄ Dialkylamino Substituenten im Rest R sind sind Methylamino, Dimethylamino, Ethylamino, Diethylamino, Propylamino, Isopropylamino, Dipropylamino, Diisopropylamino, tertiär-Butylamino. Der Rest R₂ in R kann die Bedeutung von Methyl, Ethyl, Propyl, Isopropyl, n-Butyl, Isobutyl, oder tertiär-Butyl haben. Ferner kann R auch die Bedeutung von Halogen oder Cyano haben. Der Rest R₃ in X- R₃ kann für geradkettig oder verzweigtes C₁-C₄ Alkyl, Alkenyl, oder Alkinyl stehen, wenn X = S auch für Aryl oder Heteroaryl. Beispiele für Aryl sind etwa Phenyl, 1-Naphthyl, 2-Naphthyl, für Heteroaryl etwa 2-Pyridyl, 3-Pyridyl, 4-Pyridyl, 2-Pyrimidinyl, 4-Pyrimidinyl, Pyrazinyl, Benzthiazol-2-yl.

Bevorzugte Verbindungen sind solche, in denen der Rest R₁ in A Propenyl bedeutet, B alpha-Aminobuttersäure, D N-Methyl-gamma-hydroxyleucin, E Valin, F, I, und J jeweils N-Methylleucin, G Alanin, H (D)-Alanin und K N-Methylvalin bedeuten.
In dieser Konfiguration bedeutet der Rest R in der Formel III Methyl, Ethyl, Propyl, Allyl, Propargyl, Hydroxymethyl, Hydroxyethyl, Hydroxybenzyl, Aminomethyl, Methylaminomethyl, Dimethylaminomethyl, Ethylaminomethyl, Diethylaminomethyl, Propylaminomethyl, Isopropylaminomethyl, Dipropylaminomethyl, Diisopropylaminomethyl, tertiär-Butylaminomethyl, Fluormethyl, Methoxymethyl, Ethoxymethyl, Methoxycarbonyl, Ethoxycarbonyl, Propoxycarbonyl, Isobutyloxycarbonyl, Methylaminocarbonyl, Ethylaminocarbonyl, Propylaminocarbonyl, Ispropylaminocarbonyl, Phenylaminocarbonyl, Methoxycarbonylmethyl, Ethoxycarbonylmethyl, Propoxycarbonylmethyl, tertiär-Butoxycarbonylmethyl, Methoxy, Ethoxy, Propoxy, Isopropoxy, n-Butoxy, Isobutoxy, tertiär-Butoxy, Hydroxyethoxy, Methylthio, Ethylthio, Hydroxyethylthio, Propylthio, Isopropylthio, n-Butylthio, Isobutylthio, tertiär-Butylthio, Phenylthio, 2-Pyridylthio, Benzthiazol-2-ylthio, Cyano oder Fluor.

Die erfindungsgemaessen Verbindungen können hergestellt werden, indem man aus cyclischen Peptiden der allgemeinen Formel I, in welcher D die Bedeutung von N-Methylleucin hat, mit geeigneten Basen ein Polyanion herstellt, dieses mit geeigneten Elektrophilen zur Reaktion bringt, und den neu eingeführten Rest gegebenenfalls noch weiter umwandelt, um die gewünschte Substitution der Aminosaeure C zu erhalten. Die so erhaltenen Produkte werden anschliessend durch mikrobielle Transformation hydroxyliert. Eine andere Möglichkeit zur Herstellung der erfindungsgemäßen Verbindungen besteht darin, dass man von cyclischen Peptiden der allgemeinen Formel 1, in welcher die Aminosaeure D die Bedeutung von N-Methyl-gamma-hydroxyleucin hat, wie oben beschrieben mit geeigneten Basen ein Polyanion herstellt, dieses mit geeigneten Elektrophilen zur Reaktion bringt, und den neu eingeführten Rest gegebenenfalls noch weiter umwandelt, um die gewünschte Substitution der Aminosaeure C zu erhalten. Die Herstellung der Polyanionen erfolgt,indem man eine Lösung des Edukts in einem geeigneten wasserfreien Lösungsmittel wie etwa Tetrahydrofuran bei tiefen Temperaturen, vorzugsweise bei Temperaturen unter minus vierzig Grad mit einem Überschuss einer geeigneten Base, vorzugsweise Lithium-Diisopropylamid, gelöst in wasserfreiem Tetrahydrofuran zur Reaktion bringt und das resultierende Polyanion mit einem geeigneten Elektrophil abfaengt. Dieses Verfahren entspricht im wesentlichen jenem wie von Seebach beschrieben (D. Seebach et al., Helv. Chim. Acta, Vol. 76, 1564 - 1590, 1993). Weitere geeignete Basen sind andere Alkaliamide, wie z.B. Lithium-diethylamid oder Lithiumhexamethyldisilazid sowie Alkalialkoholate wie etwa Kaliumethylat, Kalium tertiär-butylat, Kalium tertiär-amylat oder Natriumethylat. Geeignete Elektrophile sind zum Beispiel Alkylhalogenide, wie etwa Methyljodid, Ethyljodid, Allylbromid, Benzylbromid, Alkoxymethylhalogenide wie z.B. Methoxymethylchlorid, Methoxyethoxymethylchlorid, Benzyloxymethylchlorid aber auch Carbonylverbindungen, wie Aldehyde oder manche Ketone, z.B. Formaldehyd, Acetaldehyd, Benzaldehyd, Aceton, cyclische Ketone wie Cyclopropanon und dessen höhere Homologe, sowie reaktive Derivate von Kohlensäure wie Isocyanate und von organischen Carbonsäuren wie etwa Ester, z.B. Ethylformiat, Diethyloxalat, Benzoesäureetylester, Nicotinsäureethylester. Weitere in Frage kommende Elektrophile sind Halogene sowie andere Quellen von positivem Halogen wie N-Chlor-succinimid, N-Brom-succinimid, N-Fluor-N-neopentyl-p-toluolsulfonamid, Perchlorylfluorid, N-Halogencyane, wie Chlorcyan, aber auch Disulfide und deren Sulfoniumsalze, wie etwa Dimethylsulfid, Dimethylthiomethylsulfoniumjodid, Diphenyldisulfid, Dipyridyldisulfid, 2,2'-Benzthiazolyldisulfid. Die Reaktionen dieser Elektrophile führen zur Ausbildung eines neuen Chiralitätszentrums. Je nach Reaktionsbedingungen erhält man Gemische, in welchen eines der beiden möglichen Diastereomeren überwiegt. Diese neu gebildeten Diastereomeren unterscheiden sich voneinander dadurch, daß im einen Fall die Aminosäure C in der allgemeinen Formel I die (D)-Konfiguration, im anderen die (L)-Konfiguration hat. In der vorliegenden Erfindung werden jene Diastereomeren bevorzugt, in welchen der Aminosäure C in der allgemeinen Formel I die (D)-Konfiguration zukommt. Die Faktoren, welche zur bevorzugten Ausbildung des (D)-Diastereomeren oder (L)-Diastereomeren führen, sowie der Einfluß der verwendeten Basen und eine breite Reihe von Elektrophilen wurden für den Fall von Cyclosporin A als Edukt von Seebach genau untersucht, sind in der Literatur beschrieben (D.Seebach et al., Helv. Chim. Acta, Vol.76, 1564 - 1590, 1993) und wurden von anderen Autoren kritisch kommentiert (S.David, Chemtracts Organic Chemistry, Vol. 6, 303 - 306, 1993). Die in der vorliegenden Erfindung beschriebenen experimentellen Bedingungen führen überwiegend zur Ausbildung jener Diastereomeren, in welchen das neu gebildete Chiralitätszentrum der Aminosäure C die (D)-Konfiguration hat. In der vorliegenden Erfindung wurde gezeigt, daß dies auch dann zutrifft, wenn man 4-(gamma-Hydroxy)-N-methylleucin)-cyclosporin anstelle von Cyclosporin A als Edukt verwendet. Die Verwendung von 4-(gamma-Hydroxy)-N-methylleucin)-cyclosporin als Edukt für solche Transformationen ist neu und mit Gegenstand der vorliegenden Erfindung.

Auch in diesen Fällen haben die erhaltenen Produkte im allgemeinen überwiegend die Konfiguration einer (D)-Aminosäure.

Die mikrobielle Hydroxylierung der gamma-Position der Aminosaeure in Position D kann mit verschiedenen Mikroorganismen der Gattung Actinomyces durchgeführt werden, wie etwa beschrieben in Eur. J. Immunl. 1987, 17, 1359, mit *Sebekia benihana*. Die Bedeutung dieser Transformation besteht im wesentlichen darin, daß in einem Schritt die immunsuppressive Wirkung von Cyclosporin praktisch vollkommen eliminiert wird.

Die Verbindungen der Formel I besitzen bei geringer Toxizitaet interessante biologische Eigenschaften, insbesondere solche von Hemmwirkung gegen das Humane Immundefizienzvirus (HIV) und können daher als Heilmittel bei Infektionen mit HIV verwendet werden. Diese Hemmwirkung wurde in geeigneten in vitro Versuchen ab einer Konzentration von 0.05 bis 50 mg/l festgestellt. Als Heilmittel können die Verbindungen der Formel I und gegebenenfalls ihre Salze allein oder in geeigneten Arzneiformen gemeinsam mit anorganischen oder organischen pharmakologisch indifferenten Hilfsstoffen verwendet werden.

### Beispiel 1:

### Herstellung von 3-N-Methyl-(D)-serin-4-(gamma-hydroxy)-N-methylleucin)-cyclosporin.

Die Einführung einer Hydroxygruppe in die gamma-Stellung des N-Methylleucins in Position 4 von Cyclosporin A durch mikrobielle Transformation mit *Sebekia benihana* ist in Eur. J. Immunol., 1987, Vol. 17, 1359 beschrieben. Aus 3-N-Methyl-(D)serin-cyclosporin läßt sich durch diese Biotransformation 3-N-Methyl-(D)serin-4-gamma-hydroxy-N-methylleucin-cyclosporin herstellen. Der verwendete Stamm hat die Bezeichnung NRLL 11111 und gehört zur Familie der *Actinoplanaceae*.

### Startkultur:

Agarkulturen von NRLL 11111 wurden bei 27° über 10 Tage in einem sterilisierten, auf pH 7 eingestellten Medium bestehend aus 20 g Glucose, 10 g Hefeextrakt (Gistex), 10 g Pepton, 40 g Stärke, 2 g Calciumcarbonat, 40 g Agar, und 11 Wasser angezüchtet.

### Vorkultur:

Das Vorkulturmedium bestand aus 35 g Glucose, 50 g Stärke, 12.5 g Pepton, 50 g Malzextrakt, 22.5 g Hefeextrakt, 5 g Calciumcarbonat in 5 l Wasser. Ausserdem enthielt dieses Medium 5 ml einer Lösung von Spurenelementen (1 ml konz. Schwefelsäure, 5 g Eisen(II)sulfat Heptahydrat, 50 mg KJ, 100 mg Borsäure, 4 g Zinksulfat Heptahydrat, 2 g Mangan(II)chlorid Tetrahydrat, 200 mg Kupfersulfat Pentahydrat, und 2 g Kobaltchlorid Hexahydrat in 1 l destilliertem Wasser). Sporen und Mycel der Startkultur wurden in 10 ml einer 0.9%igen Salzlösung suspendiert und die Suspension zu 100 ml sterilisiertem Vorkulturmedium gegeben. Diese Kultur wurde 4 Tage bei 27° auf einem Schüttelrotor bei 200 Umdrehungen/Minute geschüttelt. Nach 4 Tagen wurde die Vorkultur mit sterilisiertem Vorkulturmedium verdünnt (1:10) und weitere 3 Tage geschüttelt.

### Hauptkultur:

Das Fermentationsmedium für die Hauptkultur (1l destillertes Wasser, 1 ml der obig beschriebenen Lösung von Spurenelementen, 200 mg Ammoniummolybdat, 255 mg Kaliumhydrogenphosphat, 120 mg Kaliumdihydrogenphosphat, 100 mg Magnesiumsulfat Heptahydrat, 50 mg Calciumchlorid Hexahydrat, 12.5 g Sojabohnenmehl, 2.5 g Hefeextrakt, 10 g Stärke, 10 g Dextrin, 10 g Cerelose) wurde auf pH 7.3 eingestellt, und 20 Minuten bei 120° sterilisiert. Dann wurden 10 ml der oben erhaltenen Vorkultur zugesetzt, die Mischung 24 Stunden bei 27° mittels eines Schüttelrotors geschüttelt und nach Zugabe von 100 mg 3-N-Methyl-(D)-serin-cyclosporin weitere 3 Tage geschüttelt.

### Isolation von 3-N-Methyl-(D)-serin-4-(gamma-hydroxy)-N-methylleucin)-cyclosporin

Die Hauptkultur wird durch Filtration vom Mycel befreit und das Filtrat 5x mit Dichlormethan extrahiert. Die vereinigten Extrakte werden in einem Rotationsverdampfer eingeengt und der Rückstand an 250 g Silicagel (Silicagel Merck, Korngröße 0.4-0.063 mesh) chromatographiert. Nochmalige Chromatographie der Cyclosporinfraktionen an 70 g Silicagel ergibt 15 mg 3-N-Methyl-(D)-serin-4-(gamma-hydroxy)-N-methylleucin)-cyclosporin als amorphes Pulver. Die optische Drehung dieser Substanz beträgt [alpha]_{D} = -166° (c = 0.5, Dichlormethan). Dieses Produkt läßt sich aus Methanol kristallisieren, wobei die erhaltenen Kristalle bei 150 - 152° schmelzen. Im ¹H-NMR Spektrum in deuteriertem DMSO erscheinen die beiden Methylgruppen des N-Methyl-gamma-hydroxyleucins als Singulette bei 1.05 ppm.

### Beispiel 2:

### Herstellung von 3-N-Methyl-(D)-serin-cyclosporin

Die Herstellung dieser Substanz ist in der Literatur beschrieben von D. Seebach, et al., Helv. Chimica Acta, Vol. 76, 1564-1590, 1993.

Diese Substanz kann auch auf folgendem Weg erhalten werden:

### 2a) 3-alpha-Methoxycarbonyl-cyclosporin

Zu einer Lösung von 8.7 ml Diisopropylamin in 800 ml trockenem THF werden bei 0°C unter Inertgas-Atmosphäre (Argon) 30 ml einer 1.6 molaren Lösung von n-Butyllithium in Hexan getropft. Nach 20 Minuten bei 0°C wird die Lösung auf minus 78°C gekühlt und portionenweise mit einer auf minus 78°C vorgekühlten Lösung von 10.0 g Cyclosporin A in 150 ml trockenem Tetrahydrofuran versetzt. Das resultierende Gemisch wird 1 Stunde bei minus 78°C gerührt, wonach ein Strom von trockenem CO₂ Gas eingeleitet wird. Anschliessend werden 6.4 ml Chlorameisensäuremethylester zugetropft und der Ansatz 2 Stunden bei minus 78°C gerührt. Nach Zugabe von 1.1 ml Diisopropylamin lässt man die Temperatur auf 20°C ansteigen, rührt das Gemisch 14 Stunden bei dieser Temperatur und erhitzt anschliessend 45 Minuten am Rückfluss. Nachdem das Gemisch auf 20°C abgekühlt ist, gibt man 100 ml 10%ige Phosphorsäure zu, extrahiert die Wasserphase mit 600 ml Essigester. Die Wasserphase wird mehrmals mit Essigster nachextrahiert. Die organischen Extrakte werden vereinigt, mit Magnesiumsulfat getrocknet und im Vakuum zur Trockne gebracht. Das resultierende Öl wird an 500 g Kieselgel (0.02-0.05 mesh) unter Verwendung eines 4:1 Gemisches von Essigester und Cyclohexan chromatographiert. Fraktionen, welche das Produkt enthalten, werden vereinigt und das Lösungsmitel im Vakuum entfernt. Man erhält so 1.9 g 3-alpha-Methoxycarbonyl-cyclosporin als farbloses Pulver vom Schmelzpunkt 138°.

### 2b) 3-N-Methyl-D-serin-cyclosporin

Zu einer Lösung von 1.0 g 3-alpha-Methoxycarbonyl-cyclosporin in 30 ml trockenem Tetrahydrofuran werden bei 0°C 2.4 ml einer 2.0 molaren Lösung von Lithiumborhydrid in THF getropft. Diese Mischung wird 22 Stunden bei 0°C gerührt. Dann werden 2 ml destilliertes Wasser zugesetzt, gefolgt von tropfenweiser Zugabe von 10%iger Salzsäure und weiteren 10 ml destilliertem Wasser. Dieses Gemisch wird mit 25 ml Essigester geschüttelt, die Phasen getrennt, und die Wasserphase noch 3x mit je 50 ml Essigester nachextrahiert. Die vereinigten Extrakte werden mit Magnesiumsulfat getrocknet und im Vakuum zur Trockne eingeengt. Der Rückstand wird an 300 g Kieselgel unter Verwendung von Essigester als Elutionsmittel chromatographiert. Die das Produkt enthaltenden Fraktionen werden vereinigt und im Vakuum eingeengt, wobei man 401 mg 3-N-Methyl-(D)-serin-cyclosporin als farbloses amorphes Pulver erhält. Der Schmelzpunkt dieses Produktes beträgt 128-130°.

### Beispiel 3:

### Herstellung von 3-N-Methyl-(D)-serin-4-(gamma-hydroxy)-N-methylleucin-cyclosporin aus 4-(gamma-hydroxy)-N-methylleucin-cyclosporin

Zu einer Lösung von 1.12 ml Diisopropylamin in 50 ml trockenem THF werden bei 0°C unter Inertgas-Atmosphäre (Argon) 5 ml einer 1.6 molaren Lösung von n-Butyllithium in Hexan getropft. Nach 20 Minuten bei 0°C wird die Lösung auf minus 78°C gekühlt und portionenweise mit einer auf minus 78°C vorgekühlten Lösung von 1.2 g 4-(gamma-hydroxy)-N-methylleucin-cyclosporin in 50 ml trockenem Tetrahydrofuran versetzt. Das resultierende Gemisch wird 1 Stunde bei minus 78°C gerührt, wonach mittels eines leichten Argon-Stroms monomerer Formaldehyd in das Reaktionsgemisch eingeleitet wird. Der Ansatz wird noch 2 Stunden bei minus 78°C gerührt. Dann läßt man die Temperatur auf 20°C ansteigen, gibt 10 ml 10%ige Phosphorsäure zu und extrahiert die Wasserphase mit 100 ml Essigester. Die Wasserphase wird mehrmals mit Essigester nachextrahiert. Die organischen Extrakte werden vereinigt, mit Magnesiumsulfat getrocknet und im Vakuum zur Trockne gebracht. Das resultierende Öl wird an 100 g Kieselgel (0.02-0.05 mesh) unter Verwendung von Essigester als Elutionsmittel chromatographiert. Fraktionen, welche das Produkt enthalten, werden vereinigt und das Lösungsmittel im Vakuum entfernt. Man erhält so 5 mg 3-N-Methyl-(D)-serin-4-(gamma-hydroxy)-N-methylleucin-cyclosporin. Die auf diesem Weg hergestellte Substanz ist nach allen physikalisch-chemischen Kriterien identisch mit der nach Beispiel 1 hergestellten Substanz.

### Test auf antivirale Aktivität:

Für diesen Test wurde die von I.Miyoshi et al. beschriebene (Nature 294, pp 770-771, 1981) T Zellinie MT4 verwendet. Der HIV-1 (Stamm IIIB) wurde während 2 Stunden bei 37° an den Zellen adsorbiert. Dann wurde das Inokulum entfernt und die infizierten Zellen auf Gewebekulturplatten gegeben, welche die Testverbindung in verschiedenen Konzentrationen enthielt. Dabei wurde das Virusinokulum so gewählt, daß bis zum vierten Tag nach der Infektion die Konzentration an viralem p24 Antigen im Zellüberstand exponentiell anstieg. Am dritten und vierten Tag nach der Infektion wurden die Zellüberstände mittels ELISA auf p24 Antigen analysiert. IC₅₀ Werte wurden durch Vergleich der p₂₄ Konzentrationen im Überstand der infizierten, mit Substanz behandelten Zellen und jener der unbehandelten infizierten Kontrolle ermittelt. Für 3-N-Methyl-(D)serin-4-gamma-hydroxy-N-methylleucin-cyclosporin wurde in diesem Test eine IC₅₀ von 0.1 mg/l ermittelt. Der entsprechende Wert für Cyclosporin A beträgt im Vergleich dazu 0.5 mg/l.

## Patentansprüche

1. Neue cyclische Peptide der allgemeinen Formel 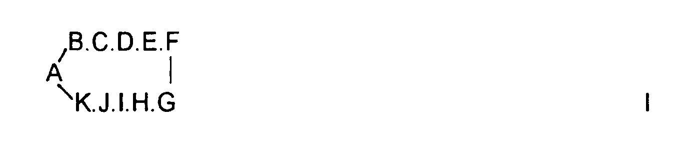 worin die Buchstaben A bis K Reste der folgenden Aminosäuren bedeuten:
A ein substituiertes Homothreonin der allgemeinen Formel
R₁-CH₂CH(CH₃)-CH(OH)-CH(NHCH₃)-COOH II
in welcher R₁ n-Propyl oder Propenyl bedeutet und die Doppelbindung vorzugsweise trans konfiguriert ist,
B alpha-Aminobuttersäure, Valin, Norvalin, oder Threonin,
C eine (D)-Aminosäure der allgemeinen Formel
CH₃NH-CH(R)-COOH III
in der
R= C₂ - C₆ Alkyl, Alkenyl, Alkinyl, geradkettig oder verzweigt, wobei diese Gruppen noch durch Hydroxy, Amino, C₁-C₄ Alkylamino, C₁-C₄ Dialkylamino, Alkoxy oder Acyloxy substituiert sein können,
COOR₂, CONHR₂, wobei R₂ = C₁-C₄ Alkyl, geradkettig oder verzweigt sein kann,
X-R₃, wobei X = O, S, und R₃ = C₁-C₄ Alkyl, Alkenyl, Alkinyl, geradkettig oder verzweigt, und wobei wenn X = S, R₃ = auch Aryl oder Heteroaryl sein kann,
Halogen, vorzugsweise Fluor,
Cyano,
CHR₄R₅, wobei R₁ = Wasserstoff, Methyl, Ethyl, Phenyl, und R₅ = Wasserstoff, Hydroxy, Halogen, vorzugsweise Fluor, weiters Amino, C₁- C₄ Alkylamino, C₁-C₄ Dialkylamino, Acyloxy, vorzugsweise Acetyloxy, weiters tert-Butoxycarbonyl-amino-ethoxy-ethoxy-acetyloxy, Alkoxycarbonyl, vorzugsweise Butoxycarbonyl,
D N-Methyl-gamma-hydroxy-leucin oder N-Methyl-gamma acetyloxyleucin
E Valin,
F, I, und J jeweils N-Methylleucin,
G Alanin,
H (D)-Alanin, (D)-Serin, und
K N-Methyl-Valin.

2. Cyclische Peptide der allgemeinen Formel I nach Anspruch 1, in welcher der Rest R₁ in A Propenyl, B alpha-Aminobuttersäure, D N-Methyl-gamma-hydroxyleucin, E Valin, F, I, und J jeweils N-Methylleucin, G Alanin, H (D)-Alanin und K N-Methylvalin bedeutet.

3. Cyclische Peptide der allgemeinen Formel I nach Anspruch 2, in welcher der Rest R in der Formel III Methyl, Ethyl, Propyl, Allyl, Propargyl, Hydroxymethyl, Hydroxyethyl, Hydroxybenzyl, Aminomethyl, Methylaminomethyl, Dimethylaminomethyl, Ethylaminomethyl, Diethylaminomethyl, Propylaminomethyl, Isopropylaminomethyl, Dipropylaminomethyl, Diisopropylaminomethyl, tertiär-Butylaminomethyl, Fluormethyl, Methoxymethyl, Ethoxymethyl, Methoxycarbonyl, Ethoxycarbonyl, Propoxycarbonyl, Isobutyloxycarbonyl, Methylaminocarbonyl, Ethylaminocarbonyl, Propylaminocarbonyl, Ispropylaminocarbonyl, Phenylaminocarbonyl, Methoxycarbonylmethyl, Ethoxycarbonylmethyl, Propoxycarbonylmethyl, tertiär-Butoxycarbonylmethyl, Methoxy, Ethoxy, Propoxy, Isopropoxy, n-Butoxy, Isobutoxy, tertiär-Butoxy, Hydroxyethoxy, Methylthio, Ethylthio, Hydroxyethylthio, Propylthio, Isopropylthio, n-Butylthio, Isobutylthio, tertiär-Butylthio, Phenylthio, 2-Pyridylthio oder Benzthiazol-2-yl-thio bedeutet.

4. 3-N-Methyl-(D)-serin-4-(gamma-hydroxy)-N-methylleucin-cyclosporin

5. Verfahren zur Herstellung von Verbindungen der allgemeinen Formel I, in welcher C die oben angegebene Bedeutung und D die Bedeutung von N-Methyl-gamma-hydroxyleucin hat, dadurch gekennzeichnet, dass man ein cyclisches Peptid der allgemeinen Formel I, in welcher D die Bedeutung von N-Methylleucin hat, mit Hilfe eines Mikroorganismus hydroxyliert.

6. Verfahren nach Anspruch 5 **dadurch gekennzeichnet, dass** der Mikroorganisus *Sebekia benihana* ist.

7. Verfahren zur Herstellung von Verbindungen der allgemeinen Formel I, in welcher D die Bedeutung von N-Methyl-gamma-hydroxyleucin hat, **dadurch gekennzeichnet, dass** man ein cyclisches Peptid der allgemeinen Formel I, in welcher C Sarcosin und D N-Methyl-gamma-hydroxyleucin bedeutet, mit geeigneten Basen in ein Polyanion überführt und dieses durch Reaktion mit geeigneten Elektrophilen in die Produkte nach Anspruch 1 umwandelt.

8. Eine pharmazeutische Zusammensetzung enthaltend ein oder mehrere cyclische Peptide nach Anspruch 1 zusammen mit einem pharmazeutisch annehmbaren Verdünnungsmittel oder Träger.

9. Verwendung einer Verbindung nach Anspruch 1 zur Herstellung eines Arzneimittels zur Behandlung oder Vorbeugung von Infektionen mit dem Humanen Immundefizienzvirus (HTV)

10. Polyanion der allgemeinen Formel I, in welcher C Sarkosin und D N-Methyl-gamma-hydroxyleucin bedeutet

11. Polyanion von 4-(gamma-hydroxy)-N-methylleucin-cyclosporin.

## Claims

1. A novel cyclic peptide of the general formula 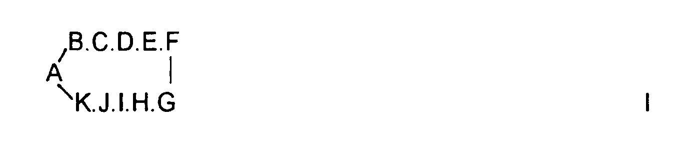 in which A to K denote residues of the following amino acids:
A is a substituted homothreonine of the general formula
R₁-CH₂CH(CH₃)-CH(OH)-CH(NHCH₃)-COOH II
in which R₁ is n-propyl or propenyl and the double bond is preferably trans-configured,
B is alpha-aminobutyric acid, valine, norvaline or threonine,
C is a (D)-amino acid of the general formula
CH₃NH-CH(R)-COOH III
in which
R is C₂-C₆ alkyl, alkenyl, alkynyl, straight-chain or branched, where these groups can additionally be substituted by hydroxyl, amino, C₁-C₄ alkylamino, C₁-C₄ dialkylamino, alkoxy or acyloxy,
COOR₂, CONHR₂, where R₂ can be C₁-C₄ alkyl, straight-chain or branched,
X-R₃, where X is O or S, and R₃ is C₁-C₄ alkyl, alkenyl or alkynyl, straight-chain or branched, and where, when X is S, R₃ can also be aryl or heteroaryl,
halogen, preferably fluorine,
cyano, or CHR₄R₅, where R₄ is hydrogen, methyl, ethyl or phenyl, and R₅ is hydrogen, hydroxyl or halogen, preferably fluorine, and, furthermore, amino, C₁-C₄ alkylamino, C₁-C₄ dialkylamino, acyloxy, preferably acetyloxy, and, furthermore, tert-butoxycarbonyl-aminoethoxy-ethoxyacetyloxy, or alkoxycarbonyl, preferably butoxycarbonyl,
D is N-methyl-gamma-hydroxyleucine or N-methyl-gamma-acetyloxyleucine,
E is valine,
F, I and J are in each case N-methylleucine,
G is alanine,
H is (D)-alanine or (D)-serine, and
K is N-methylvaline.

2. A cyclic peptide of the general formula I as claimed in claim 1, in which the radical R₁ in A is propenyl, B is alpha-aminobutyric acid, D is N-methyl-gamma-hydroxyleucine, E is valine, F, I and J are in each case N-methylleucine, G is alanine, H is (D)-alanine and K is N-methylvaline.

3. A cyclic peptide of the general formula I as claimed in claim 2, in which the radical R in the formula III is methyl, ethyl, propyl, allyl, propargyl, hydroxymethyl, hydroxyethyl, hydroxybenzyl, aminomethyl, methylaminomethyl, dimethylaminomethyl, ethylaminomethyl, diethylaminomethyl, propylaminomethyl, isopropylaminomethyl, dipropylaminomethyl, diisopropylaminomethyl, tert-butylaminomethyl, fluoromethyl, methoxymethyl, ethoxymethyl, methoxycarbonyl, ethoxycarbonyl, propoxycarbonyl, isobutyloxycarbonyl, methylaminocarbonyl, ethylaminocarbonyl, propylaminocarbonyl, isopropylaminocarbonyl, phenylaminocarbonyl, methoxycarbonylmethyl, ethoxycarbonylmethyl, propoxycarbonylmethyl, tert-butoxycarbonylmethyl, methoxy, ethoxy, propoxy, isopropoxy, n-butoxy, isobutoxy, tert-butoxy, hydroxyethoxy, methylthio, ethylthio, hydroxyethylthio, propylthio, isopropylthio, n-butylthio, isobutylthio, tert-butylthio, phenylthio, 2-pyridylthio or benzothiazol-2-yl-thio.

4. 3-N-Methyl-(D)-serine-4-(gamma-hydroxy)-N-methylleucinecyclosporin.

5. A process for preparing compounds of the general formula I, in which C has the abovementioned meaning and D has the meaning of N-methyl-gamma-hydroxyleucine, which comprises hydroxylating a cyclic peptide of the general formula I, in which D has the meaning of N-methylleucine, using a microorganism.

6. The process as claimed in claim 5, wherein the microorganism is *Sebekia benihana*.

7. A process for preparing compounds of the general formula I, in which D has the meaning of N-methyl-gamma-hydroxyleucine, which comprises converting a cyclic peptide of the general formula I, in which C is sarcosine and D is N-methyl-gamma-hydroxyleucine, into a polyanion using suitable bases and transforming this polyanion into the products as claimed in claim 1 by reacting it with suitable electrophiles.

8. A pharmaceutical composition which comprises one or more cyclic peptides as claimed in claim 1 together with a pharmaceutically acceptable diluent or excipient.

9. The use of a compound as claimed in claim 1 for preparing a pharmaceutical for treating or preventing infections with human immunodeficiency virus (HIV).

10. A polyanion of the general formula I, in which formula C is sarcosine and D is N-methyl-gamma-hydroxyleucine.

11. A polyanion of 4-(gamma-hydroxy)-N-methylleucinecyclosporin.

## Revendications

1. Nouveaux peptides cycliques de la formule générale : 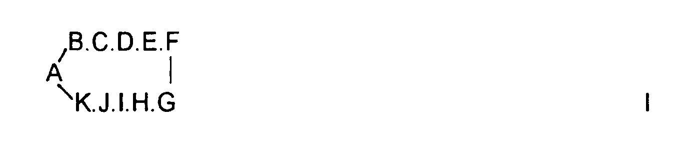 dans laquelle les lettres A à K sont des résidus des acides aminés suivants :
A homothréonine substituée de la formule générale:
R₁-CH₂-CH(CH₃)-CH(OH)-CH(NHCH₃)-COOH II
dans laquelle R₁ est un groupe n-propyle ou propényle avec la double liaison fournissant, de préférence, une structure trans,
B acide alpha-aminobutyrique, valine, norvaline ou thréonine,
C acide aminé de configuration D ayant la formule générale:
CH₃NH-CH(R)-COOH III
dans laquelle :
R= : groupe alkyle, alcényle, alcinyle en C₂ - C₆, à chaîne droite ou ramifiée, où ces groupes peuvent être substitués par des groupes hydroxy, amino, alkylamino en C₁-C₄, dialkylamino en C₁ - C₄, alcoxy ou acyloxy,
COOR₂, CONHR₂ où R₂ = groupe alkyle en C₁ - C₄, à chaîne droite ou ramifiée,
X-R₃, où X = O, S et R₃ = groupe alkyle, alcényle, alcinyle en C₁ - C₄ à chaîne droite ou ramifiée et où X = S, R₃ = aryle ou hétéroaryle, halogène (de préférence, fluor)
cyano,
CHR₄R₅, où R₄ = hydrogène, méthyle, éthyle, phényle et R₅ = hydrogène, hydroxy, halogène (de préférence fluor), ainsi qu'amino, alkylamino en C₁ - C₄, dialkylamino en C₁ - C₄, acyloxy (de préférence acétyloxy), tert-butoxycarbonyl-amino-éthoxy-éthoxy-acétyloxy, alcoxycarbonyle (de préférence butoxycarbonyle),
D N-méthyl-gamma-hydroxy-leucine ou N-méthyl-gamma-acétyloxyleucine,
E valine
F, I et J, N-méthyl-leucine
G alanine
H (D)-alanine, (D)-sérine et
K N-méthyl-valine

2. Peptides cycliques ayant la formule générale I selon la revendication 1, dans laquelle le résidu R₁ dans A est le groupe propényle, B est l'acide alpha-amino-butyrique, D est la N-méthyl-gamma-hydroxy- leucine, E est la valine, F, I et J sont la N-méthyl-leucine, G est l'alanine, H est la (D)-alanine et K est la N-méthylvaline.

3. Peptide cyclique ayant la formule générale I selon la revendication 2, dans laquelle le résidu R dans la formule III est un groupe méthyle, éthyle, propyle, allyle, propargyle, hydroxyméthyle, hydroxyéthyle, hydroxybenzyle, aminométhyle, méthylaminométhyle, diméthylaminométhyle, éthylaminométhyle, diéthylaminométhyle, propylaminométhyle, isopropylaminométhyle, dipropylaminométhyle, diisopropylaminométhyle, tert-butylaminométhyle, fluorométhyle, méthoxyméthyle, éthoxyméthyle, méthoxycarbonyle, éthoxycarbonyle, propoxycarbonyle, isobutyloxycarbonyle, méthylaminocarbonyle, éthylaminocarbonyle, propylaminocarbonyle, isopropylaminocarbonyle, phénylaminocarbonyle, méthoxycarbonyleméthyle, éthoxycarbonylméthyle, propoxycarbonylméthyle; tert-butoxycarbonylméthyle, méthoxy, éthoxy, propoxy, isopropoxy, n-butoxy, isobutoxy, tert-butoxy, hydroxy-éthoxy, méthylthio, éthylthio, hydroxyéthylthio, propylthio, isopropylthio, n-butylthio, isobutylthio, tert-butylthio, phénylthio, 2-pyridylthio, benzthiazol-2-yl-thio.

4. 3-N-méthyl-(D)-sérine-4-(gamma-hydroxy)-N-méthyl-leucine-cyclosporine

5. Procédé pour préparer des composés de la formule générale I, dans laquelle C a la signification donnée ci-dessus et D est la N-méthyl-gamma-hydroxyleucine, **caractérisé en ce que** le peptide cyclique de la formule générale 1, dans laquelle D est la N-méthyl-leucine, est hydroxylé à l'aide d'un micro-organisme.

6. Procédé selon la revendication 5, caractérisé en le micro-organisme est *Sebekia benihana.*

7. Procédé pour préparer des composés de la formule générale I, dans laquelle D est la N-méthyl-gamma-hydroxyleucine, **caractérisé en ce que** l'on convertit en un polyanion un peptide cyclique ayant la formule générale I, dans laquelle C est la sarcosine et D la N-méthyl-gamma-hydroxyleucine, en utilisant une base appropriée et **en ce que** l'on fait réagir le polyanion avec un agent électrophile approprié, pour obtenir le produit selon la revendication 1.

8. Composition pharmaceutique contenant un ou plusieurs peptides cyclique selon la revendication 1, en combinaison avec un diluant ou un vecteur acceptable sur le plan pharmaceutique.

9. Utilisation d'un composé selon la revendication 1, pour préparer une formulation thérapeutique pour un traitement préventif ou curatif d'infections par le virus de l'immunodéficience humaine (VIH).

10. Polyanion de la formule générale I, dans laquelle C est la sarcosine et D la N-méthyl-gamma-hydroxyleucine.

11. Polyanion de la 4-(gamma-hydroxy)-N-méthyl-leucine-cyclosporine.
